# EUROPEAN PATENT APPLICATION

(11) **EP 3 141 596 A1**
(43) Date of publication of application: **15.03.2017**
(21) Application number: 15789669.7
(22) Date of filing: 30.04.2015
(51) Int. Cl.: C12M 3/00, C12N 5/071, C12N 5/0783

(54) **MULTI-CHAMBER CULTURE VESSEL AND CELL CULTURING METHOD**

(30) Priority: 09.05.2014 JP 2014097377
(71) Applicant: Toyo Seikan Group Holdings, Ltd., Shinagawa-ku Tokyo 141-8627 (JP)
(72) Inventor: TANAKA, Satoshi, Yokohama-shi Kanagawa 240-0062 (JP); TOTANI, Takahiko, Yokohama-shi Kanagawa 240-0062 (JP)
(74) Representative: Liebetanz, Michael
(86) International application number: PCT/JP2015/002293
(87) International publication number: WO 2015/170466

(57) **Abstract**

To provide a culture container that can transfer cells cultured under a plurality of different culture conditions by a simple work. Provided is a multi-chamber culture container (1) obtained by processing a material having gas permeability, wherein two or more culture chambers for culturing cells are formed, each of the culture chambers (11, 12) are intercommunicated with at least of one other culture chamber and at least one external connection part (111, 121) for intercommunicating with the outside of the multi-chamber culture container is provided.

## Description

### Technical Field

The present invention relates to a technology for culturing cells, in particular, the present invention relates to a cell culture container that can culture cells under a plurality of different culture environments and culture conditions.

### Background Art

In recent years, in the fields of production of pharmaceuticals, gene therapy, immune therapy, regenerative medicine or the like, it is required to culture a large amount of cells, tissues or the like efficiently in an artificial environment.

In the process of culturing cells, tissues or the like, it may become necessary to provide a plurality of different culture environments or culture conditions. Further, cells that have been cultured in a plurality of different culture environments or culture conditions may be mixed in later steps. In these cases, the cultured cells are required to be transferred among the pluralities of containers.

However, if such cell transfer is conducted in an open system, contamination may occur. Therefore, if cell transfer is conducted manually, it is required to conduct transfer work in an aseptic environment in order to prevent such contamination. In addition, if cells are transferred among a plurality of containers, a solution may be spilled. Further, correspondence of containers has to be strictly controlled, thus making handling complicated.

Here, as a technology that can prevent contamination which occurs at the time of transferring cells or addition of a culture medium, it has been proposed to configure a closed cell culture kit by connecting a plurality of culture containers, and to conduct cell culture by using the same (see Patent Documents 1 and 2).

Patent Document 1: JP-A-2007-175028
Patent Document 2: JP-A-2005-058103

### Summary of the Invention

### Problems to be Solved by the Invention

However, even if such a cell culture kit was used, it was not possible to fully improve the complicated handling thereof.

Specifically, if such cell culture kit was used, when the number of culture containers to be connected is increased, problems arise that tubes are entangled or culture containers are jumbled to make distinction of one from another impossible, etc., thus leading to complicated handling.

Recently, a cell culture method using an automatic cell culture apparatus has been proposed. However, attachment of a cell culture kit composed of a plurality of containers to an automatic cell culture apparatus is significantly complicated.

When lymphocytes are cultured, conventionally, culture was generally conducted by a method in which lymphocytes are put in a flask to which anti-CD3 antibodies are coated, thereby to activate the lymphocytes, the activated lymphocytes are then transferred to a culture bag for amplification.

At present, technically, it has become possible to culture lymphocytes by using an automatic cell culture system by a method in which anti-CD3 antibodies are coated to the inner surface of a culture bag made of a film material having gas permeability to prepare a culture bag for cell activation, and this cell activation bag is connected to the culture bag for amplification, whereby a cell culture kit is constituted..

However, since such culture bag for activation incurs a significantly larger cost as compared with a flask, replacement of a flask with a culture bag for activation cannot be conducted easily.

Under such circumstances, the inventors of the present invention made extensive studies, and by forming a plurality of culture chambers in a single culture bag and allowing these chambers to be intercommunicated, the inventors have succeeded in producing a culture container capable of being produced at a low production cost and is significantly easy in handling. The present invention has been completed in this way.

That is, the present invention is aimed at providing a multi-chamber culture container that enables cells that have been cultured in a plurality of different culture environments or culture conditions to be transferred by a simple work and a cell culture method using the same.

### Means for Solving the Problem

In order to attain the above-mentioned object, the multi-chamber culture container of the present invention is a multi-chamber culture container obtained by processing a material having gas permeability, wherein two or more culture chambers for culturing cells are formed and each culture chamber is intercommunicated with at least one other culture chamber and is provided with at least one external connecting part for intercommunicating with the outside of the multi-chamber culture container.

The cell culture method according to the present invention is a cell culture method using the multi-chamber culture container mentioned above, wherein
as the culture chamber in the multi-chamber culture container, an activation culture chamber to which anti-CD3 antibodies for activating lymphocytes are coated and an amplification culture chamber for amplifying the activated lymphocytes are formed,
lymphocytes and a culture medium are injected into the activation culture chamber, and the lymphocytes are activated in the activation culture chamber; and
the activated lymphocytes and a culture medium are transferred from the activation culture chamber to the amplification chamber, whereby the lymphocytes are amplified in the amplification culture chamber.

Further, the cell culture method according to the present invention is a method for culturing cells using the multi-chamber culture container mentioned above, wherein
as the culture chamber in the multi-chamber culture container, an adherent cell culture chamber that separates leukocytes into lymphocytes and adherent cells to culture adherent cells, an activation culture chamber to which anti-CD3 antibodies for activating lymphocytes are coated and an amplification culture chamber for amplifying lymphocytes are formed;
leukocytes separated from blood and a culture medium are injected into the adherent cell culture chamber and the resultant is allowed to stand, and the adherent cells in the leukocytes are adhered to the bottom surface of the culture chamber for adherent cells;
lymphocytes in the leukocytes and the culture medium are transferred from the culture chamber for adherent cells to the activation culture chamber, whereby the lymphocytes are activated in the activation chamber;
a culture medium containing an inducer for dendritic cells is injected into the culture chamber for adherent cells to culture the adherent cells and the adherent cells are induced to the dendritic cells; and
the activated lymphocytes and the culture medium are transferred from the activation culture chamber to the amplification culture chamber, whereby the lymphocytes are amplified in the amplification culture chamber.

### Advantageous Effects of the Invention

According to the present invention, it is possible to provide a multi-chamber culture container capable of transferring cultured cells that have been cultured under a plurality of different culture conditions by a simple work and a cell culture method using the same.

### Brief Description of the Drawings

Fig. 1 is a view showing the multi-chamber culture container according to the first embodiment of the present invention;
Fig. 2 is a view showing the multi-chamber culture container according to the second embodiment of the present invention;
Fig. 3 is a view showing the multi-chamber culture container according to the third embodiment of the present invention;
Fig. 4 is a view showing the method for transferring the content by using the multi-chamber culture container according to the third embodiment of the present invention;
Fig. 5 is a view showing the multi-chamber culture container according to the fourth embodiment of the present invention; and
Fig. 6 is a view showing the multi-chamber culture container according to the fifth embodiment of the present invention.

### Mode for Carrying out the Invention

Hereinbelow, a detailed explanation will be made on the embodiment of the multi-chamber culture container and the cell culture method of the present invention.

### [First embodiment]

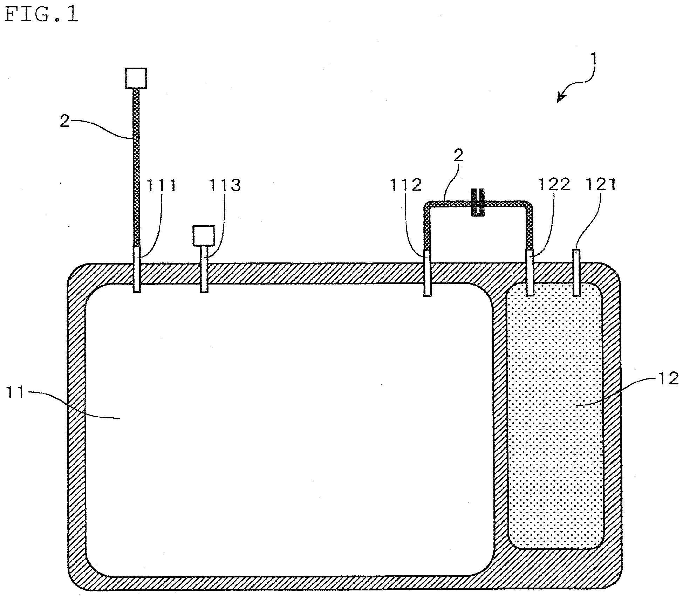

First, the multi-chamber culture container and the cell culture method according to the first embodiment of the present invention will be explained with reference to Fig. 1.

The multi-chamber culture container according to the present embodiment is a multi-chamber culture container obtained by processing a material having gas permeability, in which two or more culture chambers for culturing cells are formed and each culture chamber is intercommunicated with at least one other culture chamber, and is provided with at least one external connection part for intercommunicating with the outside of the multi-chamber culture container.

Specifically, in Fig. 1, a multi-chamber culture container 1 is provided with a culture chamber 11 and a culture chamber 12 as culture sections for culturing cells.

The culture chamber 11 is provided with an external connection port 111 for intercommunicating with the outside the multi-chamber culture container 1, a chamber-to-chamber connection port 112 for intercommunicating with other culture chambers and a sampling port 113 to conduct sampling.

Although not shown, the external connection port 111 is connected to a culture medium supply container or the like through a tube 2, and is used for supplying a culture medium to the culture chamber 11, or for other purposes.

A chamber-to-chamber connection port 112 is connected to the chamber-to-chamber connection port 122 of the culture chamber 12 through the tube 2, and is used for transferring the content liquid in the culture chamber 12 to the culture chamber 11.

The sampling port 113 is used for conducting sampling of cells cultured in the culture chamber 11.

The culture chamber 12 is provided with an external connection port 121 for intercommunicating with the outside of the multi-chamber culture container 1 and a chamber-to-chamber connection port 122 for intercommunicating with other culture chambers.

Although not shown, the external connection port 121 is connected to a cell supply container or the like through the tube 2, and is used for injection of cells to the culture chamber 12, or for other purposes.

The chamber-to-chamber connection port 122 is connected to the chamber-to-chamber connection port 112 of the culture chamber 11 through the tube 2, and is used for transferring the liquid content in the culture chamber 12 to the culture chamber 11.

The material for forming the multi-chamber culture container 1 is required to have gas permeability that is needed to culture cells. Specifically, it is preferred that the material have an oxygen permeability of 5000 ml/m²·day · atm (37°C-80%RH) or more. It is also preferred that the material for a culture chamber have transparency so that the content can be confirmed. Further, it is preferred that the material for a culture container have low cytotoxicity, low elution and suitability of radiation sterilization in order to attain high cell amplification efficiency.

As the material satisfying such requirements, a polyethylene-based resin is preferable. As the polyethylene-based resin, polyethylene, a copolymer of ethylene and α-olefin, a copolymer of ethylene and vinyl acetate, an ionomer obtained by using a copolymer of ethylene and acrylic acid or methacrylic acid and a metal ion, or the like can be given. Further, polyolefin, a styrene-based elastomer, a polyester-based thermoplastic elastomer, a silicone-based thermoplastic elastomer, a silicone resin or the like can also be used.

By subjecting such a gas-permeable material to sheet molding such as heat seal molding, pressure molding, vacuum molding or the like, the multi-chamber culture container 1 according to the present embodiment can be produced.

No specific restrictions are imposed on the shape of the multi-chamber culture container 1 of the present embodiment. However, a multi-chamber culture container having an almost rectangular shape can preferably be used. It is preferred that the shape of each culture chamber formed inside thereof be almost rectangular.

In the multi-chamber culture container 1 of the present invention, it is preferred that different culture substrates be provided in each culture chamber, thereby enabling cell culture under different culture environments and culture conditions. If there are three or more culture chambers, it is possible to provide different culture substrates respectively in all of the culture chambers or to provide different culture substrates respectively in part of culture chambers.

The application of culture by the multi-chamber culture container 1 is not particularly restricted. However, it can be preferably used in culturing lymphocytes, DC-LAK therapy (culture of dendritic cells and lymphocytes), culture of ES cells and culture of iPS cells.

Hereinbelow, cell culture when a plurality of different culture environments and culture conditions in the multi-chamber culture container 1 will be explained with reference to specific examples.

### [Culture of lymphocytes]

When culture of lymphocytes is conducted, the culture chamber 11 is used as an amplification culture chamber for amplifying lymphocytes without conducting a specific treatment on the inside thereof, and the culture chamber 12 can be used as an activation culture chamber in which an anti-CD3 antibodies are coated (coated) on the inner surface thereof, thereby to activate lymphocytes.

As shown in Fig. 1, by attaching a flow channel opening and closing means such as a clip to the tube 2 that connects the culture chamber 11 and the culture chamber 12, opening and closing of a flow channel in the tube 2 are controlled. As the flow channel opening and closing means, a pinch valve, a stopcock or the like can be used.

Then, lymphocytes and a culture medium are injected into the culture chamber 12, whereby lymphocytes are activated in the culture chamber 12. The lymphocytes that have been injected into the culture chamber 12 are activated by stimulation by anti-CD3 antibodies for about 3 days.

Thereafter, the flow channel in the tube 2 is opened, and the activated lymphocytes and the culture medium are transferred from the culture chamber 12 to the culture chamber 11, and the lymphocytes are amplified in the culture chamber 11.

At this time, in accordance with amplification of lymphocytes, the culture medium is added to the culture chamber 11. For about 4 to 10 days, activated T lymphocytes can be obtained in a large amount.

Further, it is also possible to coat other physiologically active materials on the inner surface of the culture chamber 12 to activate other cells, and transfer the activated cells to the culture chamber 11 for amplification.

### [Culture of ES cells]

When conducting culture of ES cells, the inner surface of the culture chamber 11 is subjected to a Matrigel coating treatment, and the culture chamber 11 is used as an ES cell culture chamber for amplifying ES cells. In the culture chamber 12, MEF (mouse embryonic fibroblast) that has been subjected to a mitomycin C treatment is filled, and can be used as a MEF culture chamber for culturing MEF.

Subsequently, after removing the culture medium for MEF from the culture chamber 12, the cell culture chamber 12 is washed with a culture medium for human ES cells. The culture chamber 12 is filled with the culture medium for human ES cells, and MEF is cultured overnight at 37°C at a carbon dioxide concentration of 2%. On the following day, the flow channel in the tube 2 is opened, a culture supernatant is transferred from the culture chamber 12 to the culture chamber 11. As a result, the culture chamber 11 can be used as a culture chamber provided with a MEF-conditioned culture medium.

Then, human ES cells that have been prepared in advance were seeded to the culture chamber 11, and then cultured by an MEF-conditioned culture medium (bFGF is added in advance) at 37°C at a carbon dioxide concentration of 2%, whereby human ES cells can be proliferated.

As mentioned above, by the multi-chamber culture container 1 according to the present embodiment, occurrence of contamination can be prevented. In addition, since the culture chamber 11 and the culture chamber 12 can be treated integrally, transfer of cells from the culture chamber 11 to the culture chamber 12 can be conducted quite easily.

### [Second Embodiment]

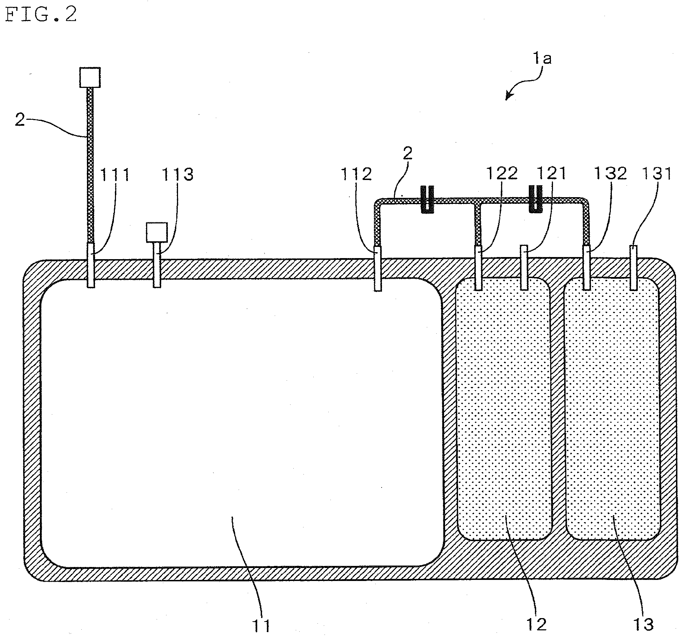

Subsequently, the multi-chamber culture container and the cell culture method according to the second embodiment of the present invention will be explained with reference to Fig. 2.

As shown in Fig. 2, a multi-chamber culture container 1a according to the present embodiment is further provided with, in addition to the culture chamber 11 and the culture chamber 12, a culture chamber 13.

The culture chamber 13 is provided with an external connection port 131 for intercommunicating with the outside of the multi-chamber culture container 1 a and a chamber-to-chamber connection port 132 for intercommunicating with other culture chambers.

Although not shown, the external connection port 131 is connected to a cell supply container or the like through the tube 2, and is used for injection of cells to the culture chamber 13 or for other purposes. The chamber-to-chamber connection port 132 is connected to the chamber-to-chamber connection port 112 of the culture chamber 11 and the chamber-to-chamber connection port 122 of the culture chamber 12 through the tube 2, and can be used for transferring the content liquid in the culture chamber 13 to the culture chamber 12 or the like. Although not shown, the external connection part 121 in the culture chamber 12 is connected to a culture medium supply container or the like through the tube 2, and can be used for supplying a culture medium to the culture chamber 12 or other purposes.

For other points, the multi-chamber culture container 1 a can have the same constitutions as those of the multi-chamber culture container 1 of the first embodiment.

In the multi-chamber culture container 1 a according to the present embodiment, by providing different culture substrates in each culture chamber, it can be used when conducting various cell cultures. For example, dendritic cells and lymphocytes can be cultured by the following method.

### [Culture of dendritic cells and lymphocytes]

The culture chamber 11 is used as an amplification culture chamber for amplifying lymphocytes without conducting a specific treatment on the inner surface thereof. The culture chamber 12 is used as an activation culture chamber for activating lymphocytes by coating on its inner surface anti-CD3 antibodies. The culture chamber 13 is used as a culture chamber for culturing adherent cells by subjecting the inner surface thereof to a treatment for culturing adherent cells. This culture chamber for adherent cells is used for culturing adherent cells by dividing leukocytes into lymphocytes and adherent cells. As shown in Fig. 2, on the tube 2 that connects the culture chamber 11 and the culture chamber 12 and on the tube 2 that connects the culture chamber 12 and the culture chamber 13, a flow channel opening and closing means such as a clip is attached, whereby opening and closing of the flow channel in the tube 2 are controlled.

To the culture chamber 13, leukocytes (cell suspension liquid) separated from blood and a culture medium are injected, and the resultant is allowed to stand at 37°C for about 2 hours. As a result, the adherent cells in leukocytes are adhered to the bottom surface of the culture chamber 13.

Subsequently, the flow channel in the tube 2 that connects the culture chamber 12 and the culture chamber 13 is opened, whereby the floating cells (lymphocytes) in leukocytes and the culture medium are transferred from the culture chamber 13 to the culture chamber 12. Then, the lymphocytes are activated in the culture chamber 12.

Subsequently, to the culture chamber 13, a culture medium (AIM-V, manufactured by Life Technology Corporation, and so on) obtained by adding 100 ng/ml of GM-CSF (Granulocytes Macrophage colony-stimulating Factor) and 50 ng/ml of IL-4 (Interleukin-4) (referred to as an inducer for dendritic cells) is added, and cultured at 37°C at a carbon dioxide concentration of 5% for about 5 days.

The immature dendritic cells induced from the adherent cells are exposed to cancer antigen, and the resultant is cultured for about 2 days, whereby mature dendritic cells can be obtained.

On the other hand, lymphocytes that have been moved to the culture chamber 12 are activated by stimulation of anti-CD3 antibodies for about 3 days.

Thereafter, the flow passage in the tube 2 that connects the culture chamber 11 and the culture chamber 12 is opened, whereby activated lymphocytes and a culture medium are transferred from the culture chamber 12 to the culture chamber 11. The lymphocytes are proliferated in the culture chamber 11. At this time, in accordance with the amplification of lymphocytes, the culture medium is added to the culture chamber 11, whereby a large amount of activated T-lymphocytes (T cells) can be obtained in about 4 days.

Finally, these cells are prepared and dendritic cells and lymphocytes can be respectively administrated to a human being.

As mentioned above, by the multi-chamber culture container 1a according to the present embodiment, if there are three or more culture chambers, by providing them in a single culture container, these three or more culture chambers can be handled integrally. As a result, transfer of cells can be conducted easily, and complicated culture steps can be conducted by simple operations.

### [Third Embodiment]

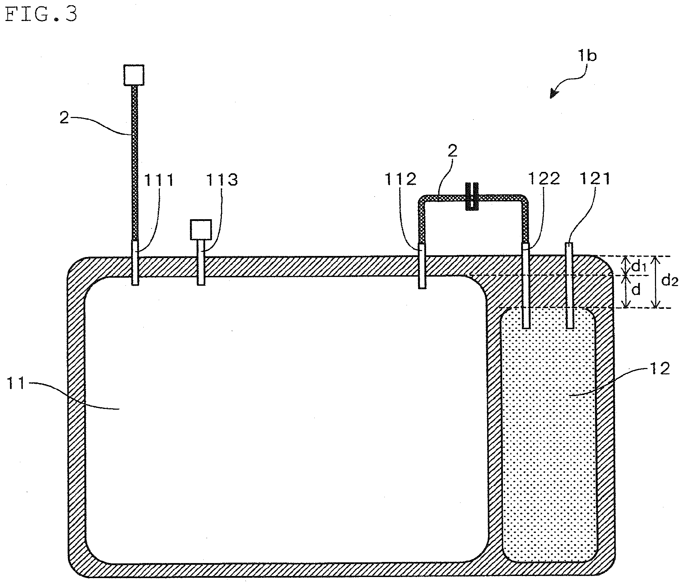

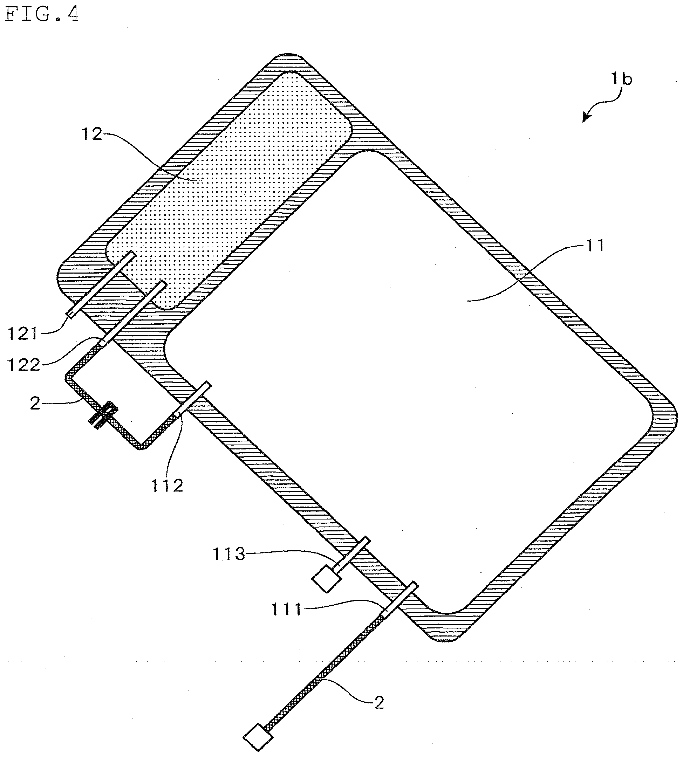

Next, the multi-chamber culture container and the cell culture method according to the third embodiment of the invention will be explained with reference to Fig. 3 and Fig. 4.

The multi-chamber culture container according to the present embodiment is characterized in that it is a multi-chamber culture container in which on one side of the multi-chamber culture container, two or more chamber-to-chamber connecting parts are provided in a state they are connected with a tube, and the culture chambers are formed such that a distance between the side in each culture chamber on which the chamber-to-chamber connecting part is provided and the one side of the multi-chamber culture container becomes large or small in the order in which these culture chambers are arranged. For other points, the constitutions can be the same as those of the multi-chamber culture container 1 of the first embodiment.

Specifically, as shown in Fig. 3, the culture chamber 11 and the culture chamber 12 are formed such that a difference between the distance d1 between the side of the multi-chamber culture container 1b on which the chamber-to-chamber connection ports 112 and 122 are provided and the side of the culture chamber 11 on which the chamber-to-chamber connection port 112 is provided and the distance d2 between the side of the multi-chamber culture container 1b on which the chamber-to-chamber connection ports 112 and 122 are provided and the side of the culture chamber 12 on which the chamber-to-chamber connection port 122 is provided becomes a prescribed value d. This d value (= d2-d1) is preferably 3 mm or more, more preferably 5 mm to 50 mm, with 7 mm to 20 mm being further preferable.

Due to such a configuration, transfer of the content from the culture chamber 12 to the culture chamber 11 can be conducted preferably only by inclining the multi-chamber culture container 1 b.

That is, the multi-chamber culture container 1 b is positioned such that a culture chamber for which a distance between the side of each of the culture chambers 11 and 12 on which the chamber-to-chamber connection ports 112 and 122 are provided and the side of the multi-chamber culture container 1 b on which the chamber-to-chamber connecting ports 112 and 122 are provided is larger is positioned higher, and the side in the multi-chamber culture container 1 b on which the chamber-to-chamber connection ports 112 and 122 are provided is positioned downwardly. Then, the multi-chamber culture container 1b is inclined such that one angle of the multi-chamber culture container 1 b is positioned at the lowest end, whereby transfer of the content from a culture chamber positioned higher to a culture chamber positioned lower is conducted.

Specifically, as shown in Fig. 4, a culture chamber 12 for which the distance between the side of the multi-chamber culture container 1 b on which the chamber-to-chamber ports 112 and 122 are provided and the side of the culture chamber on which the chamber-to-chamber connection port is provided is larger is positioned higher, and the side of the multi-chamber culture container 1 b on which the chamber-to-chamber connection parts 112 and 122 are provided is directed obliquely downward. Simultaneously, the multi-chamber culture container 1b is inclined such that one angle of the multi-chamber culture container 1b is positioned at the lowest end, whereby the content from the culture chamber 12 to be naturally transferred to the culture chamber 11 through the tube 2.

On the other hand, when the multi-chamber culture container 1 according to the first embodiment is inclined as shown in Fig. 4, a difference in pressure sufficient for transferring the content from the culture chamber 12 to the culture chamber 11 cannot be obtained, and as a result, the content remains in the culture chamber 12.

On the other hand, by the multi-chamber culture container 1 b according to the present embodiment, it is possible to significantly reduce the amount of contents remaining in the culture container 12.

### [Fourth Embodiment]

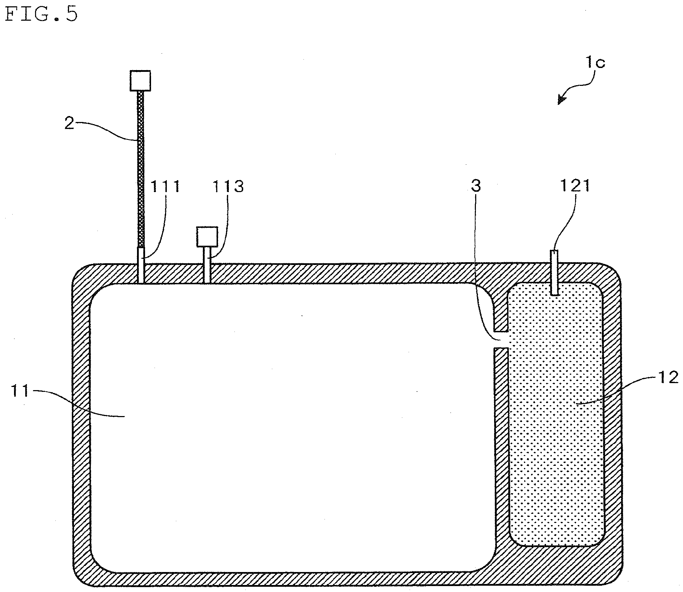

Subsequently, the multi-chamber culture container and the cell culture method according to the fourth embodiment of the invention will be explained with reference to Fig. 5.

The multi-chamber culture container according to the present embodiment is characterized in that the flow channel for intercommunicating each culture chamber with other culture chambers is formed by subjecting a film material to heat sealing.

Specifically, as shown in Fig. 5, in the multi-chamber culture container 1c, a transfer flow channel 3 intercommunicating the culture chamber 11 and the culture chamber 12 is formed. Therefore, provision of the chamber-to-chamber connection port can be omitted in the culture chamber 11 and the culture chamber 12. Other points are the same as those in the multi-chamber culture container 1 of the first embodiment.

The transfer flow channel 3 can control opening and closing by mechanical means. As such mechanical means, a pressing member or the like can be used, for example. By controlling the pressed amount, the transfer flow passage 3 can be opened and closed.

According to the multi-chamber culture container 1 c as mentioned above, it is not necessary to connect the culture chamber 11 and the culture chamber 12 by the tube 2, and provision of the chamber-to-chamber connection port in each culture chamber can be omitted, whereby it becomes possible to obtain a multi-chamber culture container having a simpler structure. Further, since no tube is provided for connecting the culture chambers, handling is very simple. Attaching the culture container to an automatic culture apparatus can be conducted significantly easily.

### [Fifth Embodiment]

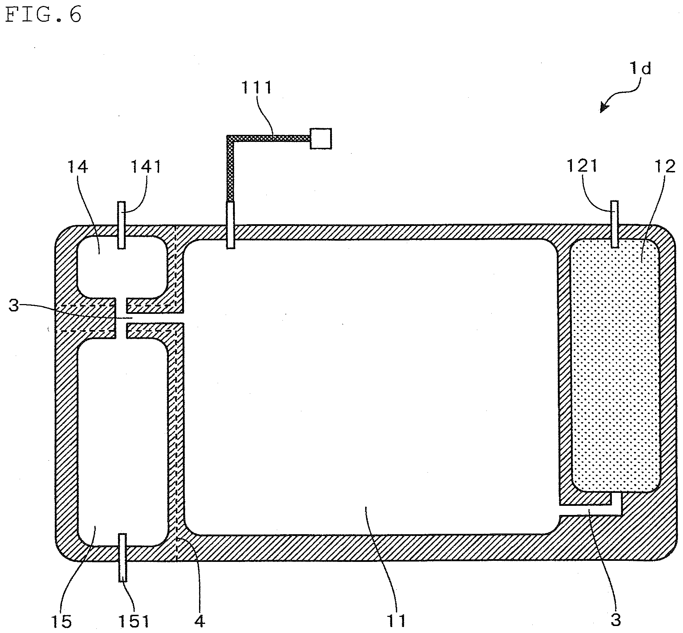

Subsequently, the multi-chamber culture container and the cell culture method according to the fifth embodiment of the invention will be explained with reference to Fig. 6.

As shown in Fig. 6, the multi-chamber culture container 1d according to the present embodiment is provided with a culture chamber 12 as an activating culture chamber for activating cells, a culture chamber 11 as an amplification culture chamber for amplifying cells, a sampling chamber 14 for sampling cells and a collecting chamber 15 for collecting cells.

Together with the formation of the transfer flow channel 3 intercommunicating the culture chamber 12 and the culture chamber 11, between the culture chamber 11 and the sampling chamber 14, as well as between the culture chamber 11 and the collecting chamber 15, the transfer flow channel 3 is formed by heat sealing a film material.

Further, an easily-cuttable part 4 composed of perforations, slits, scores, notches or the like that can separate these chambers one from another easily is provided around the sampling chamber 14 and the collecting chamber 15. Such easily-cuttable part 4 may be provided in order to separate each culture chamber.

The multi-chamber culture container 1d can be used for culture of lymphocytes by the following method, for example.

In the same manner as in the first embodiment, the culture chamber 11 is used as an amplification culture chamber for amplifying lymphocytes without conducting a specific treatment on the inner surface thereof, and the culture chamber 12 is used as an activation culture chamber for activating lymphocytes by coating anti-CD3 antibodies to the inner surface thereof.

Further, the sampling chamber 14 is used for sampling cultured cells in the culture chamber 11, and the collecting chamber 15 is used for collecting a certain amount of cultured cells in the culture chamber 11.

Then, after closing the transfer flow channel 3 intercommunicating the culture chamber 12 and the culture chamber 11 by a mechanical means, lymphocytes and a culture medium are injected into the culture chamber 12 to activate lymphocytes. Further, the transfer flow channel 3 intercommunicating the sampling chamber 14 and the culture chamber 11 and the transfer flow channel 3 intercommunicating the collecting chamber 15 and the culture chamber 11 is closed by means of a mechanical means.

After activating lymphocytes, the transfer flow channel 3 between the culture chamber 12 and the culture chamber 11 is opened, thereby to push the lymphocytes and the culture medium from the culture chamber 12 to the culture chamber 11. The activated lymphocytes are transferred to the culture chamber 11, and the lymphocytes are amplified in the culture chamber 11. At this time, in accordance with amplification of the lymphocytes, a necessary amount of a culture medium can be added from a culture medium supply container to the culture chamber 11 through the external connection port 111. Such addition of a culture medium can be conducted aseptically by using an aseptic connection apparatus or the like. Further, by improving components of a culture medium, an additional culture medium can be filled in the culture chamber 11 in advance. In addition, by forming a culture medium supply chamber in the multi-chamber culture container 1d of the present embodiment, a culture medium can be added from a culture medium supply chamber to the culture chamber 11.

When sampling cells cultured in the culture chamber 11, the transfer flow channel 3 between the sampling chamber 14 and the culture chamber 11 is opened, whereby a small amount of lymphocytes and a culture medium are transferred from the culture chamber 11 to the sampling chamber 14. Thereafter, the transfer flow channel 3 between the sampling chamber 14 and the culture chamber 11 is cut by welding by heat sealing or the like, and the sampling chamber 14 is cut off along the easily-cuttable part 4, and the cells can be used for analysis or inspection of cells.

Further, when a medical treatment method or the like in which part of cultured cells is collected and administrated it in two divided doses are conducted, the transfer flow channel 3 between the collection chamber 15 and the culture chamber 11 is opened, whereby necessary amounts of lymphocytes and a culture medium are transferred from the culture chamber 11 to the collection chamber 15. Thereafter, the transfer flow channel 3 between the collection chamber 15 and the culture chamber 11 is cut by welding by heat sealing or the like, and the collection chamber 15 is cut off along the easily-cuttable part 4, whereby lymphocytes put in the collection chamber 15 can be used for administration, or the like.

As mentioned above, according to the multi-chamber culture container 1d of the present embodiment, since all of these works can be conducted within the multi-chamber culture container 1d, cell culture can be conducted easily in a state where sterility is ensured.

The multi-chamber culture container 1d of the present embodiment is a multi-chamber culture container where flow channels are integrated. Therefore, by only designing a seal bar, various chambers or flow channels can be prepared easily at a low cost. Further, when setting in an automatic culture apparatus, since complicated works of installing tubes become unnecessary, not only work efficiency is improved but also risks such as erroneous installation can be reduced.

The present invention is not restricted to the embodiments mentioned above, and it is needless to say that various modifications are possible within the scope of the invention.

For example, various modifications are possible, e.g. combining the above-mentioned various embodiments, providing an enzyme solution supply container for supplying an enzyme solution for peeling adherent cells from the inner surface of the culture chamber in order to transfer adherent cells and providing an inhibitor solution supply container for supplying to the culture chamber an inhibitor solution for inhibiting the function of the enzyme, or the like.

### Industrial Applicability

The present invention can be preferably used when cells are cultured by using a closed culture container in the field of production of pharmaceuticals, gene therapy, regenerative medicine, immune therapy or the like.

### Description of Numerical References

- 1, 1 a, 1 b, 1 c and 1 d: Multi-chamber culture container
- 11: Culture chamber
- 111: External connection port
- 112: Chamber-to-chamber connection port
- 113: Sampling port
- 12: Culture chamber
- 121: External connection port
- 122: Chamber-to-chamber connection port
- 13: Culture chamber
- 131: External connection port
- 132: Chamber-to-chamber connection port
- 14: Sampling chamber
- 141: External connection port
- 15: Collection chamber
- 151: External connection port
- 2: Transfer tube
- 3: Transfer channel
- 4: Easily-cuttable part

## Claims

1. A multi-chamber culture container obtained by processing a material having gas permeability, wherein two or more culture chambers for culturing cells are formed and each culture chamber is intercommunicated with at least one other culture chamber and is provided with at least one external connecting part for intercommunicating with the outside of the multi-chamber culture container.

2. The multi-chamber culture container according to claim 1, wherein each culture chamber is provided with a chamber-to-chamber connection part for intercommunicating with the other culture chambers, and the chamber-to-chamber connection parts are connected by means of a tube.

3. The multi-chamber culture container according to claim 1, wherein a flow channel for allowing each of the culture chambers to be intercommunicated with the other culture containers is formed in the multi-chamber culture container by subjecting a film material having gas permeability to heat sealing.

4. The multi-chamber culture container according to any of claims 1 to 3, wherein, as the culture chamber, at least, an activation culture chamber for activating cells and an amplification culture chamber for amplifying cells are provided.

5. The multi-chamber culture container according to claim 3, that is provided with an activation culture chamber for activating cells, and an amplification culture chamber for amplifying cells as the culture chamber, a sampling chamber for sampling cells and a collection chamber for collecting cells as the culture chamber, wherein the flow channel for intercommunicating the activation culture chamber and the amplification culture chamber is formed, and a flow channel is formed in the multi-chamber culture container between the amplification culture chamber and the sampling chamber and between the amplification culture chamber and the collection chamber, respectively, by subjecting a film material having gas permeability to heat sealing.

6. The multi-chamber culture container according to claim 5, wherein an easily-cuttable part is provided for separating any chamber selected from the culture chambers, the sampling chambers and the collection chambers.

7. The multi-chamber culture container according to any of claims 1 to 6, wherein different culture substrates are provided in all or part of the culture containers.

8. The multi-chamber culture container according to any of claims 1 to 7, wherein the multi-chamber culture container is almost rectangular, and on one side of the multi-chamber culture container, two or more chamber-to-chamber connecting parts are provided in the state that they are connected with a tube, and the culture chambers are formed such that a distance between the side in each culture chamber on which the chamber-to-chamber connecting part is provided and the one side of the multi-chamber culture container becomes large or small in the order in which these culture chambers are arranged.

9. A cell culture method in which the multi-chamber culture container according to claim 8 is used, wherein a culture chamber for which a distance between a side in each culture chamber on which the chamber-to-chamber connecting part is provided and one side of the multi-chamber culture chamber is larger is positioned at a higher side, and a side in the multi-chamber culture container on which the chamber-to-chamber connection part is provided is directed obliquely downward, and the multi-chamber culture container is inclined such that one angle of the multi-chamber culture container is positioned at the lowest end and a content is transferred from the culture chamber positioned at the higher side to the culture chamber positioned at the lower side.

10. A cell culture method using the multi-chamber culture container according to any of claims 1 to 3, wherein
as the culture chamber in the multi-chamber culture container, an activation culture chamber to which anti-CD3 antibodies for activating lymphocytes are coated and an amplification culture chamber for amplifying the lymphocytes are formed,
lymphocytes and a culture medium are injected into the activation culture chamber, and the lymphocytes are activated in the activation culture chamber; and
the activated lymphocytes and a culture medium are transferred from the activation culture chamber to the amplification chamber, whereby the lymphocytes are amplified in the amplification culture chamber.

11. A cell culture method using the multi-chamber culture container according to any of claims 1 to 3, wherein
as the culture chamber in the multi-chamber culture container, an adherent cell culture chamber that separates leukocytes into lymphocytes and adherent cells to culture adherent cells, an activation culture chamber to which anti-CD3 antibodies for activating lymphocytes are coated and an amplification culture chamber for amplifying lymphocytes are formed;
leukocytes separated from blood and a culture medium are injected into the adherent cell culture chamber and the resultant is allowed to stand, and the adherent cells in the leukocytes are adhered to the bottom surface of the culture chamber for adherent cells;
lymphocytes in the leukocytes and the culture medium are transferred from the culture chamber for adherent cells to the activation culture chamber, whereby the lymphocytes are activated in the activation chamber;
a culture medium containing an inducer for dendritic cells is injected into the culture chamber for adherent cells to culture the adherent cells and the adherent cells are induced to the dendritic cells; and
the activated lymphocytes and the culture medium are transferred from the activation culture chamber to the amplification culture chamber, whereby the lymphocytes are amplified in the amplification culture chamber.
